# EUROPÄISCHE PATENTSCHRIFT

(11) **EP 1 105 370 B1**
(45) Veröffentlichungstag und Bekanntmachung des Hinweises auf die Patenterteilung: **21.04.2004**
(21) Anmeldenummer: 99940157.3
(22) Anmeldetag: 06.08.1999
(51) Int. Cl.: C07C 309/65, C07D 307/80, A61K 31/255, A61K 31/343

(54) **NEUE ARYLSULFONAMIDE UND ANALOGA**
NOVEL ARYL SULPHONAMIDES AND ANALOGUES
NOUVEAUX ARYLSULFONAMIDES ET LEURS ANALOGUES

(30) Priorität: 19.08.1998 DE 19837638
(43) Veröffentlichungstag der Anmeldung: 13.06.2001
(73) Patentinhaber: Bayer HealthCare AG, 51368 Leverkusen (DE)
(72) Erfinder: MITTENDORF, Joachim, D-42113 Wuppertal (DE); DRESSEL, Jürgen, D-42477 Radevormwald (DE); MATZKE, Michael, D-42113 Wuppertal (DE); KELDENICH, Jörg, D-42113 Wuppertal (DE); MAULER, Frank, D-51491 Overath (DE); DE VRY, Jean-Marie-Victor, D-51491 Rösrath (DE); FRANZ, Jürgen, D-58456 Witten (DE); SPREYER, Peter, D-40225 Düsseldorf (DE); VÖHRINGER, Verena, D-42113 Wuppertal (DE); SCHUMACHER, Joachim, D-42113 Wuppertal (DE); ROCK, Michael-Harold, DK-2650 Hvidovre (DK); HORVATH, Ervin, D-51373 Leverkusen (DE); FRIEDL, Arno, D-51427 Bergisch Gladbach (DE); MOHRS, Klaus-Helmut, D-42113 Wuppertal (DE); RADDATZ, Siegfried, D-51065 Köln (DE); JORK, Reinhard, D-42781 Haan (DE)
(86) Internationale Anmeldenummer: PCT/EP1999/005682
(87) Internationale Veröffentlichungsnummer: WO 2000/010967

(56) Entgegenhaltungen:
- WO-A-99/48871
- DE-A- 19 740 785
- US-A- 5 532 237

## Beschreibung

Die vorliegende Erfindung betrifft neue Arylsulfonamide und Analoga, Verfahren zu ihrer Herstellung und ihre Verwendung zur Prophylaxe und Behandlung von neurodegenerativen Erkrankungen, insbesondere zur Behandlung von Apoplexia Cerebri, Schädel-Hirn-Trauma, Schmerz und Spastizität.

Δ⁹-Tetrahydrocannabinol (Δ⁹-THC) und in geringem Maße auch Δ⁸-THC sind die biologisch aktiven Bestandteile in Extrakten der Pflanze Cannabis sativa (Marihuana, Haschisch) und sind verantwortlich für die Effekte auf das menschliche Zentrale Nervensystem (ZNS). Potentielle historische und kontemporäre therapeutische Anwendungen von Cannabis-Präparaten umfassen u.a. Analgesie, Emesis, Anorexie, Glaukom und Bewegungsstörungen.

Bislang wurden zwei Subtypen von Cannabinoid-Rezeptoren und eine Spleiß-Variante identifiziert. Der CB1-Rezeptor (Nature 1990, 346, 561) und eine Spleiß-Variante CB1a (J. Biol. Chem. 1995, 270, 3726) sind überwiegend im Zentralen Nervensystem lokalisiert. Der CB2-Rezeptor wurde überwiegend im peripheren Gewebe, insbesondere in Leukozyten, Milz und Makrophagen gefunden (Eur. J. Biochem. 1995, 232, 54).

CB1 und CB2-Rezeptoren besitzen sieben Transmembranregionen und gehören zur Familie der G-Protein-Rezeptoren. Beide Rezeptoren sind negativ gekoppelt via Gᵢ/Gₒ-Protein zur Adenylatcyclase und möglicherweise negativ gekoppelt zur präsynaptischen Freisetzung von Glutamat (J. Neurosci. 1996, 16, 4322). CB1-Rezeptoren sind darüberhinaus positiv gekoppelt mit Kalium-Kanälen sowie negativ gekoppelt mit N- und Q-Typ Calcium-Kanälen.

Vier Klassen von CB1-Rezeptor-Agonisten sind bisher bekannt: klassische Cannabinoide, wie beispielsweise Δ⁹-THC, nichtklassische Cannabinoide, Aminoalkylindole und Eicosanoide. Zu den letzten gehört der allgemein akzeptierte endogene CB1-Rezeptor-Agonist Anandamid.

Außerdem ist bekannt, daß Apoplexia Cerebri eine Folge einer plötzlichen Durchblutungsstörung eines menschlichen Gehimbereichs mit nachfolgenden Funktionsausfällen, mit entsprechenden neurologischen und/oder psychischen Symptomen ist. Die Ursachen für Apoplexia Cerebri können in Hirnblutungen (z.B nach einem Gefaßriß bei Hypertonie, Arteriosklerose und apoplektischem Aneurysma) und Ischämien (z.B. durch eine Blutdruckabfallkrise oder Embolie) liegen. Die Funktionsausfälle im Gehirn führen zu einer Degeneration oder Abtötung der Gehirnzellen (Journal of Cerebral Blood Flow and Metabolism 1981, 1, 155); Chem. Eng. News 1996 (May 13), 41; Trends Pharmacol. Sci. 1996, 17, 227). Unter Schädel/Hirn-Trauma versteht man gedeckte und offene Schädelverletzungen mit Gehirnbeteiligung.

Die vorliegende Erfindung betrifft Verbindungen der Formel

R¹-O-G-OSO₂-R² (I),

in welcher
- R¹: für einen Rest der Formel 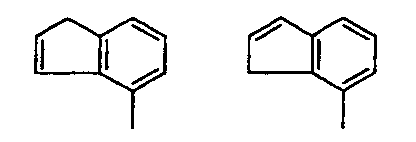
oder 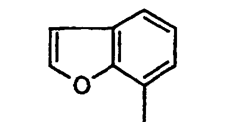 und wobei die oben aufgeführten Ringsysteme gegebenenfalls mit einem Rest ausgewählt aus der Gruppe, (C₁-C₆)-Alkoxycarbonyl, (C₁-C₈)-Alkyl, das seinerseits durch Hydroxy substituiert ist,
- G: zweifach gebundenes Phenyl, das gegebenenfalls mit einem Rest ausgewählt aus der Gruppe Hydroxy, Trifluormethyl, Fluor, Chlor, (C₁-C₃)-Alkyl, Hydroxy(C₁-C₃)alkyl, oder (C₁-C₃)-Alkoxy, substituiert ist,
- R²: (C₁-C₈)-Alkyl, das gegebenenfalls mit bis zu 3, gleichen oder verschiedenen Resten ausgewählt aus der Gruppe Fluor, Chlor, Brom, Trifluormethyl, oder Trifluormethyl-substituiertem (C₁-C₄)-Alkoxy substituiert ist, bedeuten
und deren pharmazeutisch verträgliche Salze,

Aminoschutzgruppe im Rahmen der Erfindung sind die üblichen in der Peptid--Chemie verwendeten Aminoschutzgruppen.
Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.Butoxycarbonyl, Allyloxycärbonyl, Vinyloxycarbonyl, 2-Nitrobenzyloxycarbonyl, 3,4,5-Trimethoxybenzyloxycarbonyl, Cyclohexoxycarbonyl, 1,1-Dimethylethoxycarbonyl, Adamantylcarbönyl, Phthaloyl, 2,2,2-Trichlorethoxycarbonyl, 2,2,2-Trichlor-tertbutoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomeren und Diastereomeren lassen sich in bekannter Weise in die stereoisomer einheitlichen Bestandteile trennen.

Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Zur vorliegenden Erfindung gehören auch Ammoniumverbindungen, die durch Überführung der freien Amine mittels Alkylierung hergestellt werden können.

Im Rahmen der vorliegenden Erfindung haben die Substituenten im allgemeinen die folgende Bedeutung:

(C₁-C₁₂)-Alkyl steht im allgemeinen in Abhängigkeit von den oben aufgeführten Substituenten für einen geradkettigen oder verzweigten Kohlenwassserstoffrest mit 1 bis 12 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt Bevorzugt ist (C₁-C₈)-Alkyl mit 1 bis 8 Kohlenstoffatomen, z.B. Methyl, Ethyl, Propyl, Isopropyl.

(C₂-C₁₂)-Alkenyl stehen im allgemeinen in Abhängigkeit von den oben aufgeführten Substitutenten für einen geradkettigen oder verzweigten Kohlenwasserstoffrest mit 2 bis 6 und 2 bis 20 Kohlenstoffatomen und einer oder mehreren, bevorzugt mit einer oder zwei Doppelbindungen. Bevorzugt ist der Niederalkylrest mit 2 bis 4 und 2 bis 10 Kohlenstoffatomen und einer Doppelbindung. Besonders bevorzugt ist ein Alkenylrest mit 2 bis 3 und 2 bis 8 Kohlenstoffatomen und einer Doppelbindung. Beispielsweise Isooctyl genannt. Bevorzugt ist (C₁-C₈)-Alkyl mit 1 bis 8 Kohlenstoffatomen, z.B. Methyl, Ethyl, Propyl, Isopropyl.

(C₁-C₆)-Alkoxy steht im allgemeinen in Abhängigkeit von den oben aufgeführten Substituenten für einen über ein Sauerstoffatom gebundenen geradkettigen oder verzweigten Kohlenwasserstoffrest mit 1 bis 6 Kohlenstoffatomen. Bevorzugt ist Niederalkoxy mit 1 bis 4 Kohlenstoffatomen. Besonders bevorzugt ist ein Alkoxyrest mit 1 bis 3 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

(C₁-C₆)- Alkoxycarbonyl kann beispielsweise durch die Formel 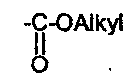 dargestellt werden.
Alkyl steht hierbei für einen geradkettigen oder verzweigten Kohlenwasserstoffrest mit 1 bis 6 Kohlenstoffatomen. Bevorzugt wird Niederalkoxycabonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Bevorzugt sind Verbindungen der Formel (I), worin
- R¹: einen Rest der Formel 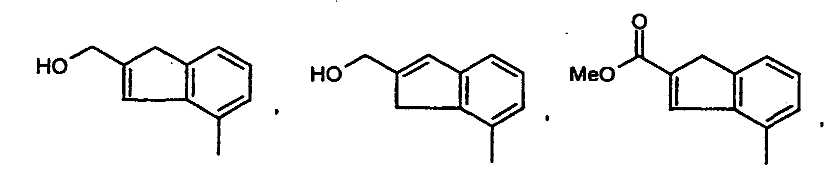 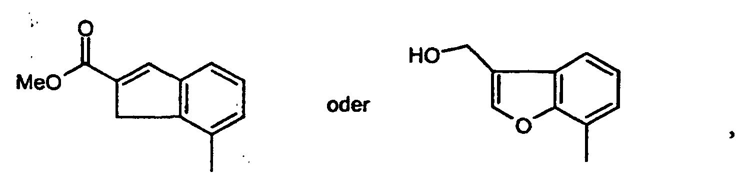
- G: zweifach gebundenes Phenyl, das gegebenenfalls mit Fluor oder Chlor substituiert ist,
- R²: (C₁-C₄)-Alkyl, das gegebenenfalls mit Fluor oder Trifluormethyl substituiert ist, bedeuten
und deren pharmazeutisch verträgliche Salze.

Ganz besonders bevorzugt sind Verbindungen ausgewählt aus der Gruppe 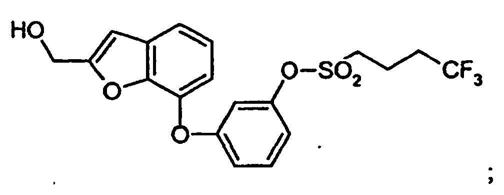 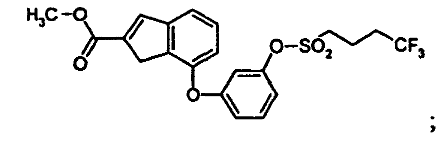 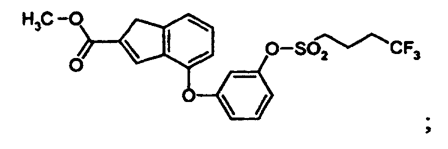 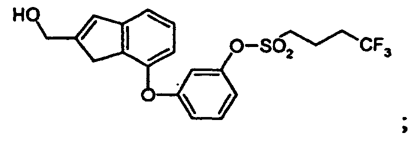 und 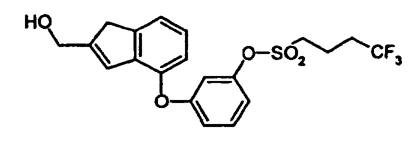 Außerdem wurde ein Verfahren zur Herstellung der erfindungsgemäßen Verbindungen der Formel (I) gefunden, dadurch gekennzeichnet, daß
[A] Verbindungen der Formel

   R¹-O-G-OH (II),

   in welcher
   - R¹, und G: die oben angegebenen Bedeutungen haben
   mit Verbindungen der Formel

   R³-SO₂ -R² (III)

   in welcher
   - R²: die im oben angegebenen Bedeutungen hat,
   - R³: für Halogen, vorzugsweise Chlor oder Iod steht,
   zu Verbindungen der Formel (I)
   in inerten Lösemitteln, gegebenenfalls in Anwesenheit einer Base, umgesetzt werden
   oder
[B] Verbindungen der Formel 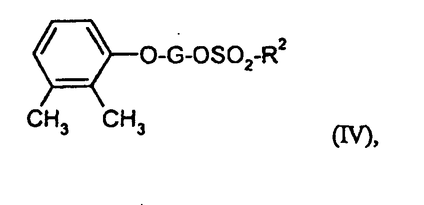
in welcher
- G und R²: die oben angegebenen Bedeutungen haben,
durch radikalische Bromierung, beispielsweise mit N-Bromsuccinimid, in einem inerten Lösungsmittel in Verbindungen der Formel 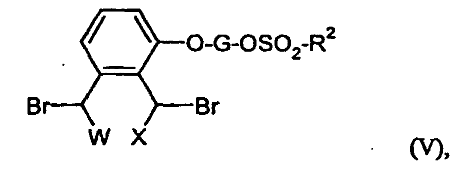 in welcher
- G und R²: die oben angegebenen Bedeutungen haben,
und einer der Substituenten W oder X für Brom und der andere für Wasserstoff steht, überführt werden,
und anschließend mit Verbindungen der Formel

CH₂(CO₂R⁴)₂ (VI),

in welchen
- R⁴: für (C₁-C₆)-Alkyl steht und
in inerten Lösemitteln, gegebenenfalls in Anwesenheit einer Base, zu Verbindungen der Formel

R⁵-O-G-OSO₂-R² (VII),

in welcher
- G und R²: die oben genannten Bedeutungen haben und
- R⁵: für einen Rest der Formel 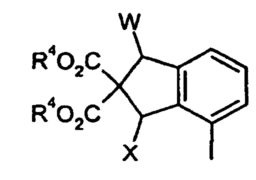 steht,
worin
R⁴ , W und X die oben genannte Bedeutung haben,
zu Verbindungen der Formel 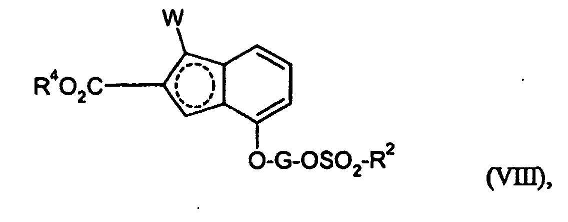 in welcher
- G, R², W und R⁴: die oben angegebenen Bedeutungen haben,
umgesetzt werden,
und abschließend eine Reduktion zu der Methylhydroxyfunktion durchführt, und gegebenenfalls in Abhängigkeit der oben aufgeführten Substituenten nach üblichen Methoden wie beispielsweise einer Alkylierung oder Veresterung Derivatisierungen anschließt,
und in einem letzten Schritt eine Reduktion mit BH₃ x S(CH₃)₂ in Tetrahydrofuran durchführt,
und im Fall der reinen Enantiomeren eine HPLC-Trennung nach üblichen Methoden durchführt,
und gegebenenfalls die oben aufgeführten Substituenten nach üblichen Methoden eingeführt und derivatisiert werden.

Die erfindungsgemäßen Verfahren können durch folgende Formelschemata beispielhaft erläutert werden:

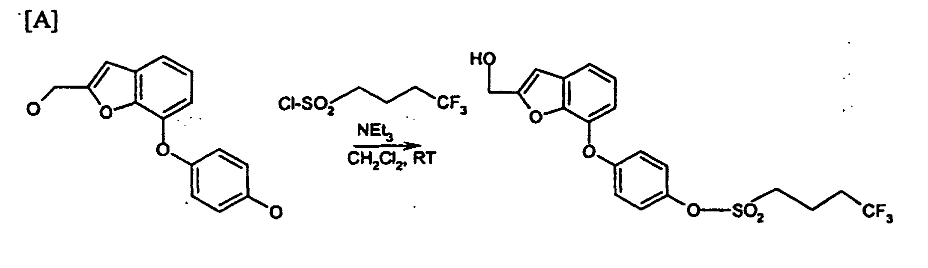

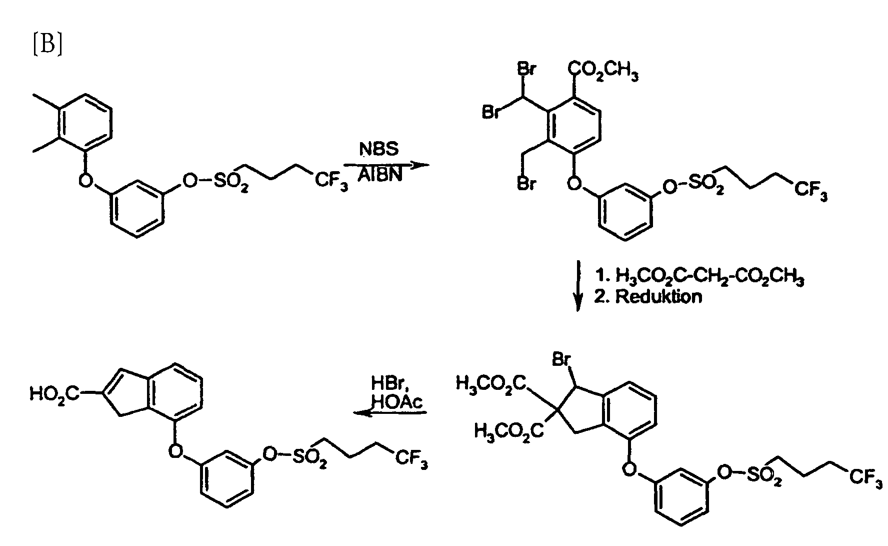

Als Lösemittel eignen sich Ether wie Diethylether, Dioxan, Tetrahydrofuran, Glykoldimethylether, oder Kohlenwasserstoffe wie Benzol, Toluol, Xylol, Hexan, Cylcohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als Basen eignen sich im allgemeine Alkalihydride oder -alkoholate, wie beispielsweise Natriumhydrid oder Kalium-tert.butylat, oder cyclische Amine, wie beispielsweise Piperidin, Pyridin, Dimethylaminopyridin oder C₁-C₄-Alkylamine, wie beispielsweise Triethylamin. Bevorzugt sind Triethylamin, Natriumhydrid, Pyridin und/oder Dimethylaminopyridin.

Als Basen eignen sich außerdem üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kaliumtert.butanolat. Besonders bevorzugt sind Kaliumcarbonat und Natriumhydroxid.

In einer Variante wird die Umsetzung in Pyridin, dem eine katalytische Menge DMAP zugesetzt wird, durchgeführt. Gegebenenfalls kann noch Toluol zugefügt werden.

Die Verfahren werden im allgemeinen bei Normaldruck durchgeführt. Es ist aber auch möglich, die Verfahren bei Überdruck oder bei Unterdruck durchzuführen (z.B. in einem Bereich von 0,5 bis 5 bar).

Die Verbindungen der Formel (II) können beispielsweise hergestellt werden, indem
[A'] Verbindungen der Formel

   R¹-R⁶ (IX)

   in welcher
   - R¹: die oben angegebenen Bedeutungen hat und
   - R⁶: für eine Abgangsgruppe, bevorzugt Halogen, besonders bevorzugt Brom, steht,
   mit Verbindungen der Formel

   HO-G-O-R⁷ (X)

   in welcher
   - G: die oben angegebenen Bedeutungen hat und
   - R⁷: für (C₁-C₆)-Alkyl, bevorzugt Methyl, steht,
   in einem inerten Lösungsmittel, bevorzugt Dimethylformamid oder Pyridin, gegebenenfalls in Anwesenheit einer Base, bevorzugt Kaliumcarbonat, und gegebenenfalls in Anwesenheit von Kupfer (I/II)-Salzen, bevorzugt Kupfer (II)-Oxid oder Kupfer (I)-Iodid, in einem Temperaturbereich von 0°C bis 200°C, bevorzugt 80 bis 150°C und Normaldruck zu Verbindungen der Formel

   R¹-O-G-O-R⁷ (I)

   in welcher
   - R¹, G und R⁷: die oben genannten Bedeutungen haben,
   umgesetzt werden und anschließend in Gegenwart einer Säure, bevorzugt Bromwasserstoffsäure, zu Verbindungen der Formel (II) reagiert werden.

In DOS 1942 264 wird die Herstellung von fluorierten Alkansulfonsäurechloriden beschrieben, in US 5 149 357 u.a. die Herstellung eines 4,4,4-Trifluorbutansulfonsäureamids, ohne jedoch die Herstellung des entsprechenden Sulfonsäurechlorids zu offenbaren.

Die fluorierten Sulfonsäurechloride wurden analog DOS 1 942 264 hergestellt.

Die Verbindungen der allgemeinen Formeln (III), (VI), (VII), (IX), (X), (XI) und (XII) sind an sich bekannt oder nach üblichen Methoden herstellbar.

Die Verbindungen der Formel (IV) sind teilweise bekannt oder neu und können durch Umsetzung der Verbindungen der Formeln (XI) und (XII) 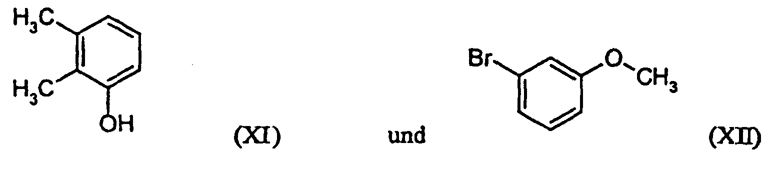 in Anwesenheit von CuO (kat.), Kaliumcarbonat und Pyridin und Verbindungen der Formel 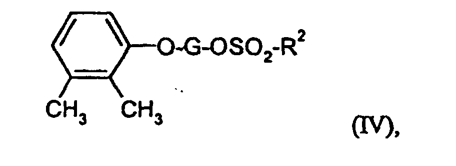 in welcher
- G und R²: die oben angegebenen Bedeutungen haben,
hergestelt werden,
und abschließend mit Bromwasserstoffsäure und Eisessig die Hydroxyfunktion freisetzt.
und/oder arteriosklerotischer Veränderungen. Zur Behandlung chronischer oder psychiatrischer Leiden wie beispielsweise Depression neurodegenerativer Erkrankungen wie beispielsweise Alzheimersche, Parkinsonsche oder Huntingtonsche Erkrankung, Multiple Sklerose, amyotrophische laterale Sklerose, Neurodegeneration durch akute und/oder chronische virale oder bakterielle Infektionen und Multiinfarktdemenz.

Darüber hinaus können sie in Arzneimitteln eingesetzt werden zur Behandlung von Schmerzzuständen, Emesis, Übelkeit, Glaukom, Asthma, Anorexie, Konvulsionen, Rheuma, Sedation und Bewegungsstörungen.

Die erfindungsgemäßen Substanzen eignen sich auch zur Behandlung von Erkrankungen, die durch bakterielle und/oder virale Infektion verursacht werden, die auf direkte und/oder indirekte Veränderungen des Immunsystems bzw. auf Fehlsteuerungen unter Mitwirkung des Immunsystems beruhen, wie z.B. bei lokalen oder systemischen Autoimmunerkrankungen (z.B. Lupus erythematodes in allen seinen Varianten), entzündlichen und/oder autoimmunologisch bedingten Erkrankungen der Gelenke (z.B. primär chronische Polyarthritis, traumatisch bedingten Entzündungen), entzündlichen und/oder autoimmunologisch bedingten Erkrankungen des Knochen- und Muskelapparates, entzündlichen und/oder autoimmunologisch bedingten krankhaften Prozessen der inneren Organe (z.B. Morbus Crohn, Glomerulonephritis) und der äußeren Organe (z.B. allergische Reaktionen durch aerogene Aufnahme von Antigenen) und des zentralen Nervensystems (z.B. Multiple Sklerose, Morbus Alzheimer, psychiatrische Erkrankungen) sowie der Sinnesorgane, primären und/oder sekundären und/oder autoimmunologischen Erkrankungen des blutbildenden Systems und des Immunsystems (z.B. Abstoßungsreaktionen, AIDS) selbst, sowie bei Hauterkrankungen entzündlicher und/oder immunologischer Genese bei Mensch und Tier. Ferner wirken diese Substanzen bei den indirekten Symptomen dieser Erkrankungen wie z.B. Schmerz.

Bevorzugt ist ihre Verwendung zur Behandlung von Schmerz, Spastizität, cerebralen Ischämien und Schädel/Him-Trauma.

Zur Löslichkeitsbestimmung wurde eine Fällungsmethode herangezogen:

10 mg der Testsubstanz werden in 50µl DMSO vollständig gelöst (Stammlösung). Von dieser Lösung gibt man 20µl in 2000µl physiologische Kochsalzlösung. Diese Lösung wiederum wird zur Equilibrierung bei 25°C im Thermomixer Comfort (Fa. Eppendorf) bei 1400 rpm 1 Stunde geschüttelt.
Die ausgefallenen Teile der Testsubstanz werden mit der Biofuge 15 Fa. Heraeus 5 min bei 14000 rpm abzentrifugiert. 1300 µl des Überstandes werden erneut mit der Microfuge Fa. Beckmann bei 45000 rpm = 125000g zentrifugiert.
10 µl dieses Zentrifugationsüberstandes werden nun mit 1000µl DMSO verdünnt und diese Lösung an der HPLC gemessen. (FA. Hewlett Packard 1090, Methode: Gradient von 100% PBS-Puffer pH=4 innerhalb von 15 min auf 10% Puffer/90% Acetonitril, Säule: RP 18)
Die gemessene Peakfläche der HPLC-Messung wird mit einer Eichgerade auf die Substanzkonzentration umgerechnet. Für die Eichgerade werden 20µl der Stammlösung sukzessiv mit DMSO so verdünnt, daß 5 Konzentrationen von 2.5 mg/l bis 2000mg/l entstehen. Diese Lösungen werden ebenfalls an der HPLC gemessen (Methode s. o.) und die Peakflächen gegen die Konzentrationen aufgetragen.

### CBI-Luciferase Reportergen Test

### 1. Klonierung des Ratten Cannabinoid Rezeptors CB1

Gesamt-RNA aus Ratten-Hirn (das Gewebe wurde frisch getöteten Tieren entnommen und in flüssigem Stickstoff schockgefroren) wurde durch saure Guanidinium-Thiocyanat/Phenol/Chloroform-Extraktion (J. Biol. Chem. 1979, 18, 5294) isoliert und mittels reverser Transkriptase und Random-Primem (jeweils von Invitrogen) in cDNA überführt. Die Polymerase Ketten Reaktion (PCR, Bedingungen: 4 min 94°C, 1x; 1 min 94°C; 2 min 53°C; 1 min 72°C, 50 Zyklen; 1 min 94°C, 2 min 53°C, 4 min 72°C, 1x) wurde in einem Perkin Elmer Thermocycler mit dem Enzym Taq Polymerase (Perkin Elmer) durchgeführt; die eingesetzten Oligonukleotid-Primer (Basen 99 bis 122: 5'→3', "down"; 1556-1575: 3'←5', "up") waren von der publizierten Sequenz des Ratten Cannabinoid- Rezeptors (Nature 1990, 346, 561) abgeleitet und wurden auf einem DNA Synthesizer, Modell 1380 der Fa. Applied Biosystems, synthetisiert. Ein Teil der PCR-Reaktion wurde in einem 1 %igen Agarose-Gel in 1x TBE-Puffer aufgetrennt und anschließend mit Ethidium-Bromid angefärbt, wobei nur eine Bande mit der erwarteten Länge sichtbar war (etwa 1,5 kb). Dieses PCR-Produkt wurde in den TA-Cloning Vektor (Invitrogen) subkloniert und die Nukleotid-Sequenz des Inserts mit T7DNA Polymerase (Sequenase, USA/Amersham) durch die Dideoxynukleotid-Kettenabbruch-Reaktion bestimmt. Das Insert besitzt eine Länge von 1477 Basenpaaren und enthält ein offenes Leseraster von 1419 Basenpaaren was einem Protein von 473 Aminosäuren entspricht. Die Anzahl der Basenpaare, die Position des offenen Leserasters und die Anzahl der Aminosäuren stimmen mit der publizierten Sequenz überein. Computer-Analysen wurden mit Hilfe der GCG Software Suite (Genetic Computer Group) durchgeführt. Das cDNA Insert wurde nach Partialverdauung mit HindIII und NotI (Biolabs) in den Expressionsvektor pRc/CMV (Invitrogen) subkloniert. Dieses Konstrukt (Plasmid CMV-RH) wurde für Transfektions-Experimente eingesetzt.

### 2. Stabile Transfektion der CHOluc9 Reporter Zellen

CHOluc9 Zellen wurden in 50 % Dulbecco's modifiziertem Eagle Medium / 50 % F-12 (DMEM/F12) gezüchtet, das 10 % foetales Kälberserum (FCS) enthielt. Transfektionen wurden in 6-well Platten angesetzt. 7,5 µg Qiagen-gereinigte CMV-RH Plasmid DNA wurde pro 105 Zellen mit dem DOTAP Transfektions System zugegeben, entsprechend dem Versuchsprotokoll des Herstellers (Boehringer Mannheim). Transfizierte Zellen wurden mit 1 mg/ml G418 selektioniert und Einzelklone wurden durch Limiting Dilution auf 96-well Platten erhalten. Zell-linien, die den Cannabinoid-Rezeptor exprimieren, wurden nach Inkubation mit dem Cannabinoid-Rezeptor Agonisten, WIN-55,212-2, in Gegenwart von Forskolin an der Hemmung der Reportergen-Expression identifiziert. Mehrere stabil transfizierte und subklonierte Zellinien wurden mittels RT-PCR, wie unter 1. beschrieben, weiter charakterisiert.

### 3. Test-Optimierung und pharmakologische Charakterisierung der CHOCB1 Reporter-Zellinie

Der Luciferase-Test wurde mit dem Ziel hoher Sensitivität und Reproduzierbarkeit, geringer Varianz und guter Eignung für die Durchführung auf dem Robotersystem optimiert durch Variation mehrerer Testparameter, wie z.B. Zelldichte, Dauer der Anzuchtphase und der Testinkubation, Forskolin-Konzentration, Medium-Zusammensetzung. Zur pharmakologischen Charakterisierung der Zellen und zum Robotergestützten Substanz-Screening wurde das folgende Testprotokoll verwendet: Die Stammkulturen wurden in 50 % Dulbecco's modifiziertem Eagle Medium / 50 % F-12 (DMEM/F12) mit 10 % FCS bei 37°C unter 10 % CO₂ gezüchtet und jeweils nach 2 bis 3 Tagen 1:10 gesplittet. Testkulturen wurden mit 5000 Zellen pro Napf in 96-well Platten ausgesät und 70 Stunden bei 37°C angezogen. Dann wurden die Kulturen vorsichtig mit Phosphat-gepufferter Saline gewaschen und mit serumfreiem Ultra-CHO Medium (Bio-Whittaker) rekonstituiert. Die in DMSO gelösten Substanzen wurden 1 x in Medium verdünnt und zu den Testkulturen pipettiert (maximale DMSO-Endkonzenträtion im Testansatz: 0,5 %). 20 Minuten später wurde Forskolin zugegeben und die Kulturen anschließend 3 Stunden im Brutschrank bei 37°C inkubiert. Danach wurden die Überstände entfernt und die Zellen durch Zugabe von 25 µl Lysereagens (25 mM Triphosphat, pH 7,8 mit 2mM DTT, 10 % Glycerin, 3 % TritonX100) lysiert. Direkt danach wurde Luciferase Substrat Lösung (2,5mM ATP, 0,5 mM Luciferin, 0,1 mM Coenzym A, 10mM Tricin, 1,35mM MgSO4, 15mM DTT, pH 7,8) zugegeben, kurz geschüttelt, und die Luciferase-Aktivität mit einem Hamamatzu Kamerasystem gemessen.

Zur Inaktivierung von Gᵢ-Proteinen wurden die Testkulturen vor dem Test für 16 Stunden mit 5 ng/ml (Endkonz.) Pertussis Toxin behandelt.

Die IC₅₀-Werte wurden mit dem Programm GraphPadPrism berechnet (Hill-Gleichung, speziell: one-site competition).

| Aktivität im Ratten CB1-Rezeptor-Luciferase Rezeptorgen Test | |
|---|---|
| **Beispiel** | **IC**_{**50**} **(nmol/l)** |
| 1 | 0,55 |

### hCB2-Luciferase Reportergen Test

CHOluc9 Zellen wurden mit dem humanen CB2-Rezeptor stabil transfiziert. Transfektion, Klonselektion und Testentwicklung wurden analog zu den Arbeiten mit dem Ratten CB1-Rezeptor durchgeführt. Das folgende Testprotokoll wurde zur pharmakologischen Charakterisierung der Zellen und zur Substanz-Testung verwendet:

Die Stammkulturen wurden in 50% Dulbecco's modifizierten Eagle Medium/50% F-12 (DMEM/F12) mit 10% FCS bei 37°C unter 10% CO₂ gezüchtet und jeweils nach 2 bis 3 Tagen 1:10 gesplittet. Testkulturen wurden mit 5000 Zellen pro Napf in 96-well-Platten in DMEM/F12 Medium mit 5 % FCS ausgesät und 70 Stunden bei 37°C angezogen. Dann wurde das Medium von den Kulturen entfernt und durch serumfreies Ultra-CHO Medium (Bio-Whittaker) ersetzt. Die in DMSO gelösten Substanzen (200x Endkonzentration) wurden zu den Testkulturen pipettiert (maximale DMSO-Endkonz. im Testansatz: 0,5%) und 20 min später wurde Forskolin zugegeben. Anschließend wurden die Kulturen 3,5 Stunden im Brutschrank bei 37°C inkubiert. Danach wurden die Überstände entfernt und die Zellen durch Zugabe von 25 µl Lysereagens (25 mM Trisphosphat, pH 7,8 mit 2 mM DTT, 10 % Glycerin, 3 % Triton X100) lysiert. Direkt anschließend wurden 50 µl Luciferase Substrat Lösung, doppelt konzentriert, (5 mM ATP, 1 mM, Luciferin, 0,2 mM Coenzym A, 10 mM Tricin, 1,35 mM MgSO₄, 15 mM DTT, pH 7,8) zugegeben, kurz geschüttelt, und die Luciferase-Aktivität mit einem Photomultiplier-Kamera-Meßsystem (Hamamatzu) bestimmt.

Die IC₅₀-Werte wurden mit dem Program GraphPad Prism™ berechnet (Hill-Gleichung; speziell: one site competition).

### Bindungsstudien an Ratten Cortex Membranen

Membranprotein wird nach Standardmethoden aus unterschiedlichen Geweben bzw. von Zellen präpariert. Puffer, markierter Ligand, DMSO oder Teststubstanz werden zusammenpipettiert, anschließend werden 100 µg Protein hinzugegeben, die Mischung gut vermischt und 60 min bei 30°C im Wasserbad inkubiert. Nach Ablauf der Inkubationszeit wird die Reaktion durch Zugabe von eiskaltem Inkubationspuffer in jedes Röhrchen gestoppt. Nach Abfiltrieren wird mit 3/4 ml Inkubationspuffer nachgewaschen. Die Filter werden in Minivials überführt, die Radioaktivität wird in einem Flüssigszintillationszähler bestimmt.

### Inhibition der Glutamat-Freisetzung

Nach Dekapitieren einer Ratte wird der Schädel eröffnet, das Gehirn herausgehoben und entlang der Mittelfurche durchschnitten. Der Hippocampus wird freipräpariert, vom restlichen Gewebe getrennt, in 350 µM dicke Schnitte geschnitten und für 60 min in Siebgefäßen bei 37°C inkubiert. Gefolgt von Basalwert und Stimulation 1 mit 75 mM KCl (S1) werden die Schnitte mit Testsubstanz inkubiert und dann die Stimulation mit KCl und Testsubstanz (S2) wiederholt. Die Glutamat-Konzentration der zu untersuchenden Proben wird dann über eine enzymatische Reaktion (GLDH) und fluorometrischer Messung von NADH gemessen. Anhand einer Eichkurve wird der Glutamatgehalt der Probe bestimmt, und unter Kenntnis des Proteingehaltes kann der Glutamatgehalt/mg Protein errechnet werden. Verglichen wird das Verhältnis S2/S1, Glutamat-Freisetzungsinhibitoren reduzieren dieses Verhältnis konzentrationsabhängig.

Mit der folgenden Testmethode kann die *in vitro*-Umwandlung der erfindungsgemäßen Aminosäureester in die entsprechenden Alkohole bestimmt werden.

### Bestimmung der Stabilität von Substanzen im Blut verschiedener Spezies (Ratte, Hund, Human)

### Prinzip der Methode

Die Testsubstanz wird in heparinisiertem Blut jeder Testspezies inkubiert. Zu geeigneten Zeitpunkten werden Aliquote des Ansatzes entnommen und in eine Acetonitrilvorlage pipettiert. Nach Zentrifugation wird der Überstand eingedampft und der Rückstand in einem für die Analytik geeigneten Lösungsmittel aufgenommen.

### Material

| | |
|---|---|
| Laborzentrifuge: | Sigma 4K10 (Sigma Laborzentrifugen, Osterode, Germany) |
| Schüttler: | KS500 (Janke und Kunkel, IKA Labortechnik, Staufen, Germany) |
| Wasserbad, Thermomix® | 1442D (Braun-Melsungen, Melsungen, Germany) |
| Abdampfvorrichtung | BAYER AG |

### Durchführung

Zur Bestimmung der Stabilität einer Testsubstanz *in vitro* wird die Substanz, die in einem kleinen Volumen eines geeigneten Lösungsmittels gelöst ist, in einer Konzentration von z.B.
2 µg/ml in 5 ml Blut bei 37 °C über 5 Stunden inkubiert. Zu geeigneten Zeitpunkten werden 1 00 µl des Ansates zu 500 µl Acetonitrilvorlage pipettiert und gemischt. Nach Zentrifugation bei 3000 rpm wird der Überstand entnommen und in einem Wasserbad bei
40 °C zur Trockne eingedampt. Der Rückstand wird in einem für die Analytik geeigneten Lösungsmittel aufgenommen.

| | |
|---|---|
| Lösungsmittel: | 10 µl EtOH / 5 ml Blut |
| Schüttlergeschwindigkeit: | 250 rpm |
| Zentrifugation | 3000 rpm |
| Zentrifugationszeit: | 10 min |
| Blutvolumen: | 5 ml |
| Blutaliquote: | 100 µl |
| Inkubationszeiten: | 0, 2, 5, 10, 15, 30, 45 Minuten, 1, 2, 3, 5 Stunden |

### Pharmakokinetik der Substanzen in der Ratte

### 1. Intravenöse Infusion

Die Substanz wird über einen Venenkatheter (Introcan®, 22G1, Braun, Melsungen, Germany) über eine laterale Schwanzvene direkt in den Blutstrom infundiert. Für die exakte Verabreichung der gewählten Dosis und des Volumens wird eine kalibrierte 10 ml Spritze verwendet. Für die Infusion wird die Pumpe Nr.54021 0 von TSE, Bad Homburg, FRG benutzt.

### 2. Probenahme und Aufarbeitung

### Blut und Plasma

Blutproben werden von katheterisierten Tieren (Vena jugularis) in heparinisierten Röhrchen gesammelt. Das Blut wird zentrifugiert und das Plasma auf geeignete Weise für die Analytik vorbereitet. Das Plasma wird bis zur Analytik bei < -15 °C aufbewahrt.

### Pharmakokinetik der Substanzen im Hund

### 1. Intravenöse Infusion

Nach Kannülierung einer oberflächlichen Vene am Vorder- oder Hinterlauf wird die Substanz direkt in den Blutstrom infundiert. Der Venenkatheter (z.B. Introcan® 20 G / 1¼, B. Braun, Melsungen, Germany) wird mit einer kalibrierten Spritze, welche an der Infusionspumpe befestigt ist, verbunden.

### 2. Probenahme und Aufarbeitung

### Blut und Plasma

Blutproben werden durch Punktion einer oberflächlichen Vene am Vorder- oder Hinterlauf oder einer Jugularvene entnommen. Die für die Infusion benutzte Extremität wird für die Blutentnahme nicht verwendet. Das Blut wird zentrifugiert und das Plasma bis zur Analytik bei < -15 °C aufbewahrt.

### Hypothermie

### 1. Agonismus Prüfung:

Fünf Minuten nach Bestimmung der Basal-Körpertemperatur via Oesophagus Temperatursonde wird die Prüfsubstanz (i.v.) appliziert. Eine Kontrollgruppe erhält, ebenfalls i.v., nur das Lösungsmittel der Prüfsubstanzen. Die Körpertemperatur wird 7,5, 15, 30 und 60 Minuten nach i.v.-Applikation gemessen. Die Gruppengröße pro Dosis beträgt 5-7 Tiere (Ratten).

### 2. Antagonismus Prüfung:

60 Minuten vor Prüfsubstanz Applikation wird der spezifische CB1 Antagonist *SR 141716A*, der Kontrollgruppe nur das Lösemittel (Solutol/0,9% NaCl) intraperitoneal appliziert. Die basale Körpertemperatur wird fünf Minuten vor Applikation von *SR 141716A* via Oesophagus Temperatursonde gemessen. Das weitere Vorgehen entspricht der Methode "Agonismus Prüfung". Die Gruppengröße pro Dosis beträgt 5-7 Tiere (Ratten).

### Permanente focale cerebrale Ischämie bei der Ratte (MCA-O)

Unter Isofluran Anästhesie wird die Arteria cerebri media einseitig freipräpariert mittels Elektrokoagulation diese und deren Nebenäste irreversibel verschlossen. Als Folge des Eingriffs entsteht ein cerebraler Infarkt. Während der Operation wird die Körpertemperatur des Tieres auf 37°C gehalten. Nach Wundverschluß und Abklingen der Narkose werden die Tiere wieder in ihren Käfig entlassen. Die Substanzapplikation erfolgt nach unterschiedlichen zeitlichen Schemata und über unterschiedliche Applikationswege (i.v, i.p.) nach der Okklusion. Die Infarktgröße wird nach 7 Tagen bestimmt. Dazu wird das Gehirn entnommen, histologisch aufgearbeitet und mit Hilfe eines computergestützten Auswertsystemes das Infarktvolumen bestimmt.

### Subdurales Hämaton bei der Ratte (SDH)

Unter Anästhesie wird den Tieren einseitig subdural Eigenblut injiziert. Unter dem Hämatom bildet sich ein Infarkt. Die Substanzapplikation erfolgt nach unterschiedlichen zeitlichen Schemata und über unterschiedliche Applikationswege (i.v., i.p.). Die Bestimmung der Infarktgröße erfolgt wie beim Modell der Permanenten focalen Ischämie bei der Ratte (MCA-O) beschrieben.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös.

Im allgemeinen hat es sich als vorteilhaft erwiesen, bei intravenöser Applikation Mengen von etwa 0,01 bis 10 mg/kg, vorzugsweise etwa 0,1 bis 10 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

### Beispiel 1A

### Thiocyansäure-4,4,4-trifluorbutylester

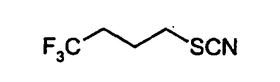

Eine gerührte Lösung von 4,4,4-Trifluorbutanol (35 g; 0,027 mol) und Triethylamin (28,3 g; 0,280 mol) in 200 ml Dichlormethan wurde bei 0°C tropfenweise mit einer Lösung von Methansulfonsäurechlorid (32,1 g; 0,280 mol) in 100 ml Dichlormethan versetzt. Nach Ende der Zugabe wurde weitere 30 min gerührt, dann auf Eis gegossen und anschließend die Phasen getrennt. Die organische Phase wurde über Magnesiumsulfat getrocknet und unter vermindertem Druck aufkonzentriert. Es wurden 55 g rohes 4,4,4-Trifluorbutyl-methansulfonat als oranges Öl erhalten.

Das Mesylat (55 g) wurde mit Natriumthiocyanat (30,6 g; 0,30 mol) in Aceton (300 ml) 6 h unter Rückfluß gekocht. Nach Abkühlung auf Raumtemperatur wurde die Mischung auf Eis gegossen, die Phasen getrennt und die organische über Magnesiumsulfat getrocknet. Nach Filtration und Aufkonzentrieren unter vermindertem Druck wurden 41 g (89 % d.Th.) Thiocyansäure-4,4,4-trifluorbutylester als Öl erhalten.
¹⁹F-NMR (376 MHz, CDCl₃; CFCl₃) d [ppm]: -66,3
¹H-NMR (400 MHz, CDCl₃, TMS) d [ppm]: 2,15 (m, 2H); 2,3 (m, 2H); 3,05 (t, J = 7,1 Hz, 2H)

### Beispiel 2A

### 4,4,4-Trifluorbutansulfonsäurechlorid

F₃C-CH₂-CH₂-CH₂-SO₂Cl

In eine Lösung von Beispiel 1 A (40 g; 0,236 mol) in wäßriger Essigsäure (150 ml Essigsäure und 70 ml Wasser) wurde bei 20°C bis 40°C Chlor eingeleitet und der Fortschritt der Reaktion gaschromatographisch verfolgt. Als die Chlorierung vollständig war, wurde der Überschuß Chlor mittels Durchleitung eines Stickstoffstromes verdrängt, 200 ml Wasser zugefügt und die Reaktionsmischung mit Dichlormethan mehrfach extrahiert. Die vereinigten organischen Phasen wurden über Magnesiumsulfat getrocknet, davon abfiltriert und unter vermindertem Druck aufkonzentriert. Man erhielt 44 g (89 % d.Th.) 4,4,4-Trifluorbutansulfonsäurechlorid als gelbes Öl.
¹⁹F-NMR (376 MHz, CDCl₃; CFCl₃) d [ppm]: -66,65 (t, J = 10 Hz)
¹H-NMR (400 MHz, CDCl₃, TMS) d [ppm]: 3,8 (m, 2H); 2,35 (m, 4H)

### Beispiel 3A

### 7-Hydroxy-2-hydroxymethyl-benzofuran

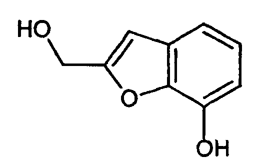

Eine Lösung von 2-Hydroxymethyl-7-methoxy-benzofuran (2,94 g; 16,5 mmol; Herstellung WO 96 20925) in N-Methylpyrrolidon (45 ml) wird mit wasserfreiem Natriumsulfid (6,89 g; 88,3 mmol) versetzt und 48 h bei 160°C unter Argon gerührt. Die Reaktionsmischung wird nach dem Abkühlen in 300 ml 2 N HCl gegossen und mit Ethylacetat (3 x 150 ml) extrahiert. Die vereinten organischen Phasen werden getrocknet (Na₂SO₄) und i.V. eingeengt. Der Rückstand wird an Kieselgel mit Toluol/Ethylacetat (1:1) chromatographiert.
Ausbeute: 1,83 g (68% d. Th.)
Smp.: 136-138°C
R_{f} = 0,36 (Toluol/Ethylacetat = 1:1)
MS (DCI/NH₃): m/z = 182 (M+NH₄)

### Beispiel 4A

### 2-Hydroxymethyl-7-(3-methoxyphenyloxy)-benzofuran

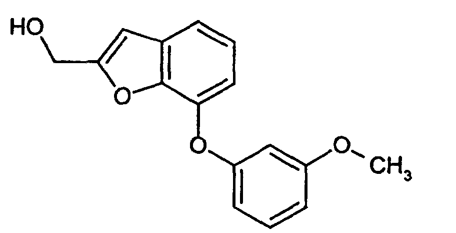

Beispiel 3A (1,85 g; 11,3 mmol) 3-Bromanisol (12,65 g; 67,6 mmol) und Kaliumcarbonat (3,12 g; 22,5 mmol) werden in Pyridin (60 ml) unter Argon vorgelegt und unter Rühren auf 140°C erhitzt. Nach Zugabe von Kupfer-(I)-iodid (2,15 g; 11,3 mmol) wird der Ansatz 27 h bei 140°C gerührt. Nach dem Abkühlen wird über Kieselgur filtriert, mit Dichlormethan (150 ml) gewaschen und i.V. eingeengt. Der Rückstand wird in Ethylacetat (200 ml) und Wasser (200 ml) aufgenommen und der entstandene Niederschlag wird abgesaugt und verworfen. Nach Phasentrennung wird die organische Phase mit 1 N HCl (2 x 200 ml) und Wasser (200 ml) gewaschen, getrocknet (Na₂SO₄) und i.V. eingeengt. Der Rückstand wird an Kieselgel mit Toluol/Ethylacetat (5:1) chromatographiert.
Ausbeute: 2,05 g (67% d. Th.)
R_{f} = 0,30 (Toluol/Ethylacetat = 5:1)
MS (DCI/NH₃): m/z = 288 (M+NH₄)

### Beispiel 5A

### 2-Hydroxymethyl-7-(3-hydroxyphenyloxy)-benzofuran

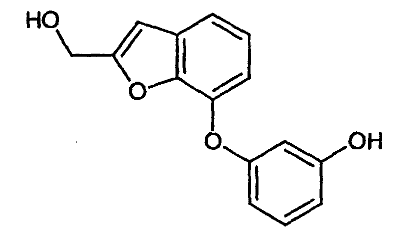

Die Herstellung erfolgte in Analogie zur Herstellung des Beispiels 3A ausgehend von Beispiel 4A (1,95 g; 7,21 mmol).
Ausbeute: 0,465 g (25% d. Th.)
R_{f} = 0,47 (Toluol/Ethylacetat = 1:1)
MS (ESI): m/z = 279 (M+Na)

### Beispiel 6A

### 3-(2,3-Dimethylphenyloxy)-anisol

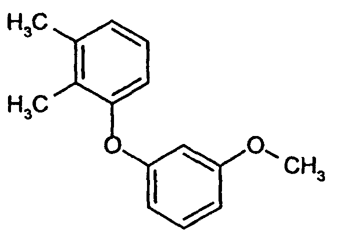

2,3-Dimethyl-1-brombenzol (80,0 g; 0,432 mol), 3-Methoxyphenol (107,3 g; 0,865 mol) und Kaliumcarbonat (119,5 g; 0,865 mol) werden unter Argon in Pyridin (350 ml) vorgelegt und auf 100°C erhitzt. Nach Zugabe von Kupfer-(II)-oxid (51,6 g; 0,648 mol) wird der Ansatz bei 140°C gerührt. Nach 15 h und 40 h wird nochmals 2,3-Dimethyl-1-brombenzol (80,0 g; 0,432 mol nach 15 h und 66,0 g; 0,357 mol nach 40 h) zugegeben. Nach 64 h wird der Ansatz i.V. eingeengt, der Rückstand in Ethylacetat aufgenommen und mit halbkonz. Salzsäure auf pH 2-3 eingestellt. Nach Phasentrennung wird die organische Phase mit ges. NaCl-Lösung gewaschen, getrocknet (Na₂SO₄) und i.V. einrotiert. Der Rückstand wird an Kieselgel mit Tol:EE = 5:1 chromatographiert.
Ausbeute: 94,9 g (36% d. Th.)
R_{f} = 0,76 (Toluol)
MS (DCI/NH₃): m/z = 246 (M+NH₄)

### Beispiel 7A

### 3-(2,3-Dimethylphenyloxy)-phenol

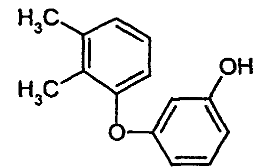

Beispiel 6A (109,6 g; 480 mmol) wird in 48 % wäßrigem Bromwasserstoff (900 ml) und Essigsäure (1500 ml) vorgelegt und über Nacht unter Rückfluß gerührt. Anschließend wird der Ansatz im Vakuum eingeengt, der Rückstand in Wasser aufgenommen und dreimal mit Ethylacetat extrahiert. Die vereinten organischen Phasen werden zweimal mit Wasser gewaschen, getrocknet (MgSO₄) und im Vakuum eingeengt. Der Rückstand wird an Kieselgel mit Toluol:EE (10:1) chromatographiert.
Ausbeute: 86,5 g (83 % d.Th.)
R_{f} = 0,15 (Toluol)
MS (ESI): m/z = 215 (M+H)

### Beispiel 8A

### 4,4,4-Trifluor-1-butansulfonsäure-3-(2,3-dimethylphenyloxy)-phenylester

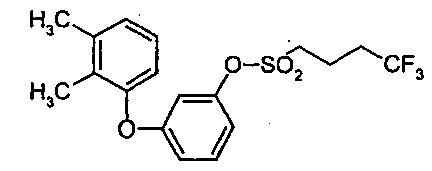

Die Herstellung erfolgt in Analogie zur Herstellung des Beispiels 1 ausgehend von Beispiel 7A (4,54 g; 21,2 mmol).
Ausbeute: 7,80 g (95% d.Th.)
R_{f} = 0,51 (Toluol)
MS (DCI / NH₃): m/z = 406 (M+NH₄)

### Beispiel 9A

### 4,4,4-Trifluor-1-butansulfonsäure-3-(2-brommethyl-3-dibrommethylphenyloxy)-phenylester

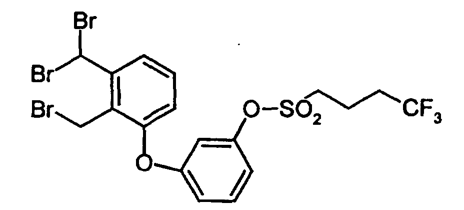

Eine Lösung von Beispiel 8A (6,76 g; 17,4 mmol) in Tetrachlorkohlenstoff (150 ml) wird mit N-Bromsuccinimid (6,50 g; 36,5 mmol) versetzt, auf Rückfluß erhitzt und unter Rühren 5 h mit einer 300 W-Lampe bestrahlt. Nach dem Abkühlen wird ausgefallenes Succinimid abgesaugt und das Filtrat im Vakuum eingeengt. Der Rückstand wird an Kieselgel mit Toluol chromatographiert. Man erhält ein Gemisch (ca. 5:1) von 4,4,4-Trifluor-1-butansulfonsäure-3-(2,3-bis-brommethylphenyloxy)-phenylester und dem gewünschten Produkt (HPLC, Nucleosil C18, Acetonitril, 0,01 M H₃PO₄). Das so erhaltene Gemisch wurde ohne weitere Reinigung weiter verwendet.

### Beispiel 10A

### (R,S)-4,4,4-Trifluor-1-butansulfonsäure-3-(1-Brom-2,2-bis-methoxycarbonylindanyl-4-oxy)-phenylester

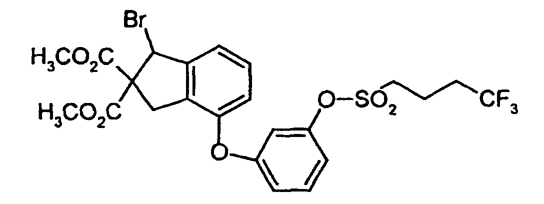

Das in Beispiel 9A erhaltene ca. 5.1-Gemisch von 4,4,4-Trifluor-1-butansulfonsäure 3-(2,3-bis-brommethylphenyloxy)-phenylester und 4,4,4-Trifluor-1-butansulfonsäure-3-(2-brommethyl-3-dibrommethylphenyloxy)-phenylester (6,00 g) wird in 2-Butanon (150 ml) gelöst. Nach Zugabe von Malonsäuredimethylester (1,136 g; 8,6 mmol) und Kaliumcarbonat (5,35 g; 38,7 mmol) wird das Reaktionsgemisch über Nacht unter Rückfluß gerührt. Nach dem Abkühlen werden die nicht gelösten Salze abgesaugt und das Filtrat i.V. eingeengt. Der Rückstand wird an Kieselgel mit Toluol : Ethylacetat (20: 1) chromatographiert. Als Hauptprodukt wird 4,4,4-Trifluor-1-butansulfonsäure-3-(2,2-bis-methoxycarbonyl-indanyl-4-oxy)-phenylester (1,95 g; 35% d. Th., erR_{f} = 0,45 (Toluol/Ethylacetat =20:1)) erhalten.
Ausbeute an Beispiel 10A: 0,82 g (16% d. Th.)
R_{f} = 0,52 (Toluol/Ethylacetat = 20:1)
MS (DCI/NH₃): m/z = 612, 614 (M+NH₄)

### Herstellungsbeispiele

### Beispiel 1

### 2-Hydroxymethyl-7-[3-(4,4,4-trifluorbutyl-1-sulfonyloxy)phenyl-1-oxy]-benzofuran

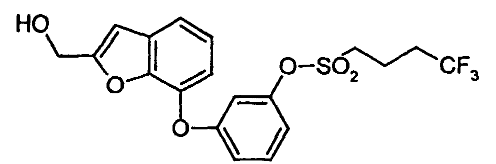

Eine Lösung von Beispiel 5A (0,382 g; 1,49 mmol) in Dichlormethan (10 ml) wird bei RT unter Argon mit Beispiel 2A (0,314 g; 1,49 mmol) versetzt und 1 h bei RT gerührt. Nach Zugabe von Triethylamin (0,151 g; 1,49 mmol) wird weitere 48 h bei RT gerührt. Anschließend wird die Reaktionsmischung mit Wasser (2 x 50 ml) gewaschen, getrocknet (Na₂SO₄) und i.V. eingeengt. Der Rückstand wird an Kieselgel mit Toluol/Ethylacetat (10:1) chromatographiert.
Ausbeute: 0,292 g (45% d. Th.)
R_{f} = 0,67 (Toluol/Ethylacetat = 1:1)
MS (DCI/NH₃): m/z = 448 (M+NH₄)

### Beispiele 2 und 3

### 4,4,4-Trifluor-1-butansulfonsäure-3-(2-methoxycarbonyl-indenyl-4-oxy)-phenylester und 4,4,4-Trifluor-1-butansulfonsäure-3-(2-methoxycarbonyl-indenyl-7-oxy)-phenylester

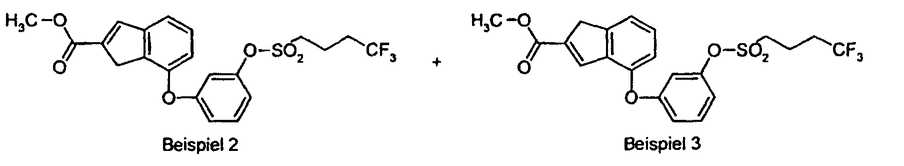

Eine Lösung von Beispiel 10A (0,904 g; 1,52 mmol) in Essigsäure (9 ml) und Bromwasserstoff, 48% ig in Wasser (3 ml) wird 24 h unter Rückfluß gerührt. Anschließend wird der Ansatz im Vakuum eingeengt, der Rückstand in Ethylacetat (50 ml) aufgenommen und mit Wasser (3 x 50 ml) gewaschen. Die vereinten organischen Phasen werden über Na₂SO₄ getrocknet und i.V. eingeengt. Der Rückstand wird in Dichlormethan (8 ml) gelöst und bei -10°C unter Argon mit Methanol (0,243 g, 7,60 mmol), N-Ethyl-N'-3-(dimethylaminopropyl)-carbodiimid Hydrochlorid (0,321 g; 1,67 mmol) und 4-Dimethylaminopyridin (0,019 g; 0,15 mmol) versetzt und über Nacht bei RT gerührt. Der Ansatz wird mit Wasser, zweimal mit ges. wäßriger NaHCO₃-Lösung und mit Wasser gewaschen, getrocknet (Na₂SO₄) und i.V. eingeengt. Der Rückstand wird an Kieselgel mit Toluol/Ethylacetat (10:1) chromatographiert.
Ausbeute: 0,357 mg (51% d. Th. eines ca. 2:1-Gemisches des Beispiel 2 und 3)
R_{f} = 0,40 (Toluol/Ethylacetat = 10:1)
¹H-NMR (CDCl₃): δ = 7,72 (t, J = 0,5 Hz, 1-CH des Indenylsubstituenten von
Beispiel 2), 7,69 (t, J = 0,5 Hz, 1-CH des Indenylsubstituenten von Beispiel 3)
MS(DCI/NH₃): m/z = 474 (M+NH₄)

### Beispiele 4 und 5

### 4,4,4-Trifluor-1-butansulfonsäure-3-(2-hydroxymethyl-indenyl-4-oxy)-phenylester (Beispiel 4) und 4,4,4-Trifluor-1-butansulfonsäure-3-(2-hydroxymethyl-indenyl-7-oxy)-phenylester (Beispiel 5)

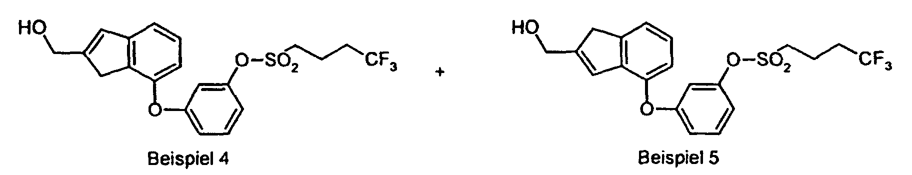

Zur Lösung des 2:1-Gemisches der Beispiele 2 und 3 (283 mg, 0,62 mmol) in Dichlormethan (10ml) tropft man bei -70°C unter Argon Diisobutylaluminiumhydrid, 1 M in Dichlormethan (1,55 ml; 1,55 mmol) und läßt 45 min bei -70°C rühren. Anschließend wird der Ansatz auf -10°C erwärmt und mit Methanol (1 ml) und einer ges. wäßrigen Natrium-Kalium-Tartrat-Lösung (20 ml) versetzt. Die Wasserphase wird mit Dichlormethan (2 x 50 ml) extrahiert und die vereinten organischen Phasen werden getrocknet (Na₂SO₄) und i.V. eingeengt. der Rückstand wird an Kieselgel mit Toluol/Ethylacetat = 2:1 chromatographiert. Man erhält ein ca. 2:1-Gemisch der Beispiele 4 und 5 (R_{f} = 0,56, Toluol/Ethylacetat = 1:1, MS (DCI/NH₃): m/z = 446 (M+NH₄)). Dieses Gemisch (120 mg) wird mittels präparativer HPLC (Daicel Chiralpak AD, 10µm, 250 x 20 mm, Fluß 6 ml/Min., Laufmittel 35% n-Heptan und 65% Ethanol, Detektion 260 nM, T = 40°C) in die Regioisomeren Beispiel 4 und Beispiel 5 getrennt.

### Beispiel 4:

Ausbeute: 54 mg
Retentionszeit: 4,01 min
¹H-NMR (D6-DMSO): δ = 3,2 (2H; 3-CH₂ vom Indanylsubstituenten), 6,71 (1H; 1-CH vom Indanyl) ppm

### Beispiel 5:

Ausbeute: 32 mg
Retentionszeit: 4,54 min
¹H-NMR (D6-DMSO): δ = 3,48 (2H; 3-CH₂ vom Indanylsubstituenten), 6,50 (1H; 1-CH vom Indanylsubstituenten)

## Patentansprüche

1. Verbindungen der Formel
R¹-O-G-OSO₂-R² (I),
in welcher
R¹ einen Rest der Formel 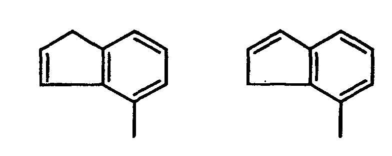 oder 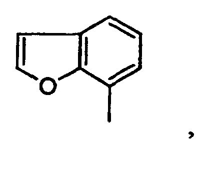
und wobei die oben aufgeführten Ringsysteme gegebenenfalls mit einem Rest ausgewählt aus der Gruppe (C₁-C₆)-Alkoxycarbonyl oder (C₁-C₈)-Alkyl, das seinerseits durch Hydroxy substituiert ist,
G zweifach gebundenes Phenyl, das gegebenenfalls mit einem Rest ausgewählt aus der Gruppe Hydroxy, Trifluormethyl, Fluor, Chlor, (C₁-C₃)-Alkyl, Hydroxy(C₁-C₃)alkyl oder (C₁-C₃)-Alkoxy substituiert ist,
R² (C₁-C₈)-Alkyl, das gegebenenfalls mit bis zu 3 gleichen oder verschiedenen Resten ausgewählt aus der Gruppe Fluor, Chlor, Brom, Trifluormethyl oder Trifluormethyl-substituiertem (C₁-C₄)-Alkoxy substituiert ist, bedeuten
und deren pharmazeutisch verträgliche Salze.

2. Verbindungen der Formel (I), gemäß Anspruch 1
in welcher
R¹ einen Rest der Formel 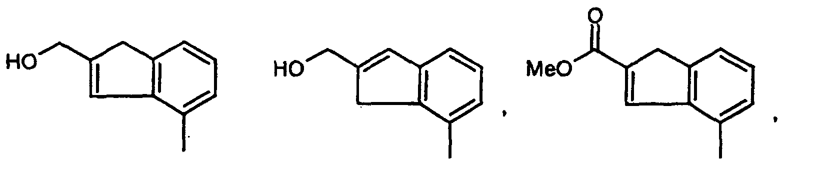 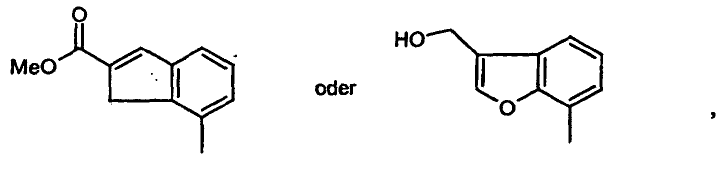
G zweifachgebundenes Phenyl, das gegebenenfalls durch Fluor oder Chlor substituiert ist,
R² (C₁-C₄)-Alkyl, das gegebenenfalls durch Fluor oder Trifluormethyl substituiert ist, bedeuten
und deren pharmazeutisch verträgliche Salze.

3. Verbindungen nach Anspruch 1, ausgewählt aus der Gruppe 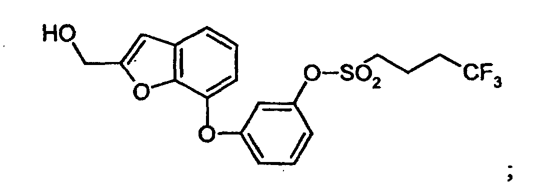 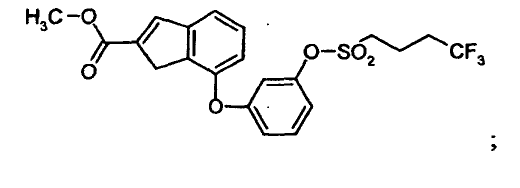 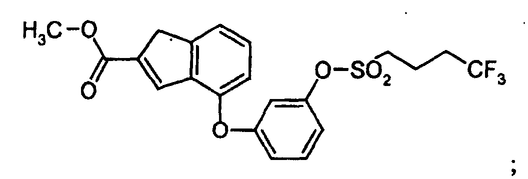 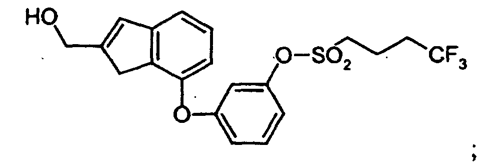 und

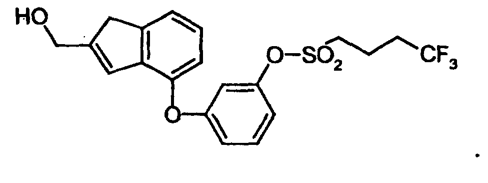

4. Verfahren zur Herstellung von neuen Arylsulfonsäureestern gemäß Ansprüchen 1 bis 3 **dadurch gekennzeichnet, daß** man
[A] Verbindungen der Formel
R¹-O-G-OH (II),
in welcher
R¹ und G die oben angegebenen Bedeutungen aufweisen
mit Verbindungen der Formel
R³-SO₂-R² (III),
in welcher
R² die im Anspruch 1 angegebenen Bedeutungen aufweist,
R³ für Halogen, vorzugsweise Chlor oder Iod steht,
zu Verbindungen der Formel (I) in inerten Lösemitteln, gegebenenfalls in Anwesenheit einer Base, umgesetzt werden
oder
[B] Verbindungen der Formel 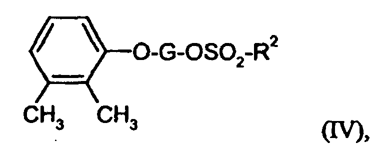
in welcher
G und R² die oben angegebenen Bedeutungen afuweisen,
durch radikalische Bromierung, beispielsweise mit N-Bromsuccinimid, in einem inerten Lösungsmittel in Verbindungen der Formel 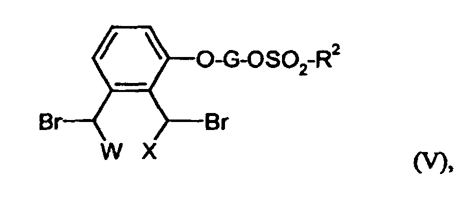 in welcher
G und R² die oben angegebenen Bedeutungen aufweisen,
und einer der Substituenten W oder X für Brom und der andere für Wasserstoff steht,
überführt werden,
und anschließend mit Verbindungen der Formel
CH₂(CO₂R⁴)₂ (VI),
in welchen
R⁴ für (C₁-C₆)-Alkyl steht und
in inerten Lösemitteln; gegebenenfalls in Anwesenheit einer Base, zu Verbindungen der Formel
R⁴-O-G-OSO₂-R² (VII),
in welcher
G und R² die oben angegebenen Bedeutungen aufweisen und
R⁴ für einen Rest der Formel 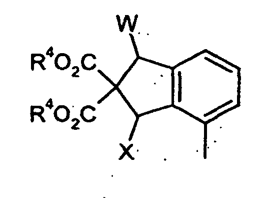 steht,
worin
R⁴ , W und X die oben angegebenen Bedeutungen aufweisen,
zu Verbindungen der Formel 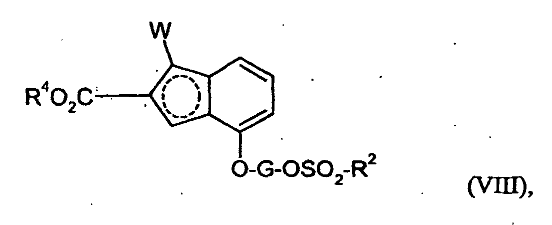 in welcher
G, R², W und R⁴ die oben angegebenen Bedeutungen aufweisen,
umgesetzt werden,
und abschließend eine Reduktion zu der Methylhydroxyfunktion durchführt,
oder
und gegebenenfalls in Abhängigkeit der oben aufgeführten Substituenten nach üblichen Methoden wie beispielsweise einer Alkylierung oder Veresterung Derivatisierungen anschließt,
und in einem letzten Schritt eine Reduktion mit BH₃ x S(CH₃)₂ in Tetrahydrofuran durchführt,
und im Fall der reinen Enantiomeren eine HPLC-Trennung nach üblichen Methoden durchführt,
und gegebenenfalls die oben aufgeführten Substituenten nach üblichen Methoden eingeführt und derivatisiert werden.

5. Pharmazeutische Zubereitungen, die als aktiven Bestandteil mindestens eine Verbindung gemäß irgendeinem der Ansprüche 1 bis 3 in Zusammenmischung mit mindestens einem pharmazeutisch verträglichen im wesentlichen nichtgiftigen Träger oder Exzipienten umfaßt.

6. Verbindungen nach irgendeinem der Ansprüche 1 bis 3 zur Verwendung als Medikament in der Behandlung von Menschen und Tieren.

7. Verwendung der Verbindungen gemäß irgendeinem der Ansprüche 1 bis 3 für die Herstellung eines Medikamentes zur Prävention und/oder Behandlung neurodegenerativer Erkrankungen.

8. Verwendung der Verbindungen gemäß irgendeinem der Ansprüche 1 bis 3 für die Herstellung eines Medikamentes zur Prävention und/oder Behandlung von cerebralen Ischämien und Schädel/Him-Trauma.

9. Verwendung der Verbindungen gemäß irgendeinem der Ansprüche 1 bis 3 für die Herstellung eines Medikamentes zur Behandlung von Schmerzzuständen, Emesis, Übelkeit, Glaukom, Asthma, Anorexie, Konvulsionen, Rheuma, Sedation und Bewegungsstörungen.

10. Verwendung der Verbindungen gemäß irgendeinem der Ansprüche 1 bis 3 für die Herstellung eines Medikamentes zur Behandlung von bakteriellen oder viralen Infektionen, Autoimmunerkrankungen, entzündlicher oder autoimmunologisch bedingter Erkrankungen der Gelenke des Knochen- und Muskelapparates, der inneren und äußeren Organe, des zenralen Nervensystems, der Sinnesorgane und des blutbildenden Systems bei Mensch und Tier.

11. Verwendung der Verbindungen gemäß irgendeinem der Ansprüche 1 bis 3 für die Herstellung eines Medikamentes zur Behandlung von Migräne und Spastizität.

## Claims

1. Compounds of the formula
R¹-O-G-OSO₂-R² (I)
in which
R¹ represents a radical of the formula 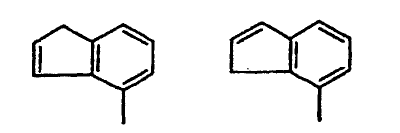 or 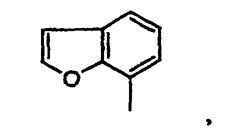
where the ring systems shown above are optionally substituted by a radical selected from the group consisting of (C₁-C₆)-alkoxycarbonyl and (C₁-C₈)-alkyl which for its part is substituted by hydroxyl,
G represents doubly attached phenyl which is optionally substituted by a radical selected from the group consisting of hydroxyl, trifluoromethyl, fluorine, chlorine, (C₁-C₃)-alkyl, hydroxy-(C₁-C₃)-alkyl or (C₁-C₃)-alkoxy,
R² represents (C₁-C₈)-alkyl which is optionally substituted by up to 3 identical or different radicals selected from the group consisting of fluorine, chlorine, bromine, trifluoromethyl and trifluoromethyl-substituted (C₁-C₄)-alkoxy,
and their pharmaceutically acceptable salts.

2. Compounds of the formula (I) according to Claim 1
in which
R¹ represents a radical of the formula 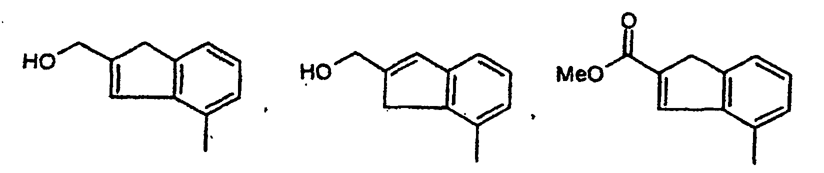 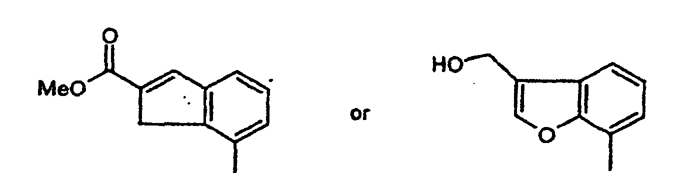
G represents doubly attached phenyl which is optionally substituted by fluorine or chlorine,
R² represents (C₁-C₄)-alkyl which is optionally substituted by fluorine or trifluoromethyl,
and their pharmaceutically acceptable salts.

3. Compounds as claimed in Claim 1, selected from the group consisting of 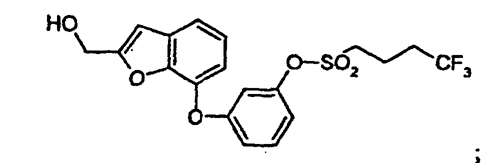 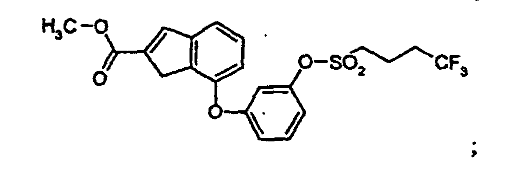 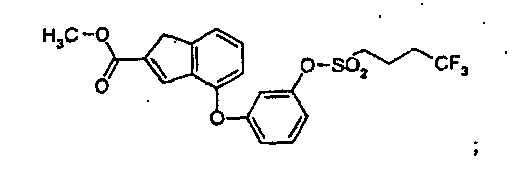 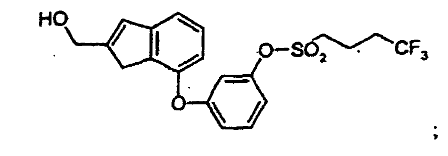 and

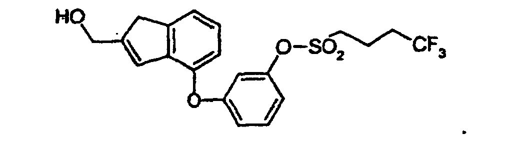

4. Process for preparing novel arylsulphonic acid esters as claimed in any of Claims 1 to 3, **characterized in that**
[A] compounds of the formula
R¹-O-G-OH (II)
in which
R¹ and G are as defined above
are reacted in inert solvents, if appropriate in the presence of a base, with compounds of the formula
R³-SO₂-R² (III)
in which
R² is as defined in Claim 1,
R³ represents halogen, preferably chlorine or iodine,
to give compounds of the formula (I)
or
[B] compounds of the formula 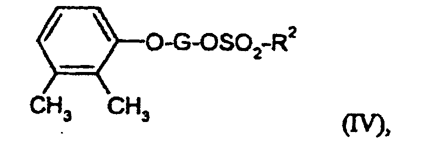
in which
G and R² are as defined above
are converted by free-radical bromination, for example with N-bromosuccinimide, in an inert solvent into compounds of the formula 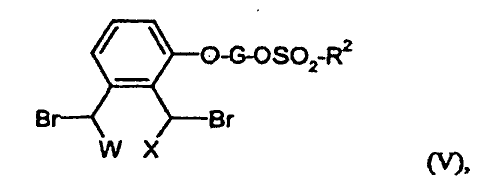 in which
G and R² are as defined above,
and one of the substituents W and X represents bromine and the other represents hydrogen,
and then, in inert solvents, if appropriate in the presence of a base, reacted with compounds of the formula
CH₂(CO₂R⁴)₂ (VI)
in which
R⁴ represents (C₁-C₆)-alkyl,
to give compounds of the formula
R⁵-O-G-OSO₂-R² (VII)
in which
G and R² are as defined above and
R⁵ represents a radical of the formula 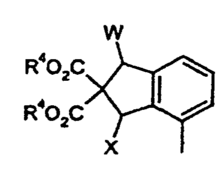 in which
R⁴, W and X are as defined above,
which are then converted into compounds of the formula 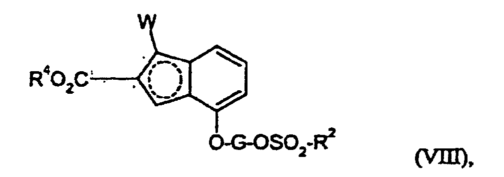 in which
G, R², W and R⁴ are as defined above,
and finally reduced to the methylhydroxy function,
followed, if appropriate and depending on the substituents listed above, by derivatizations by customary methods such as, for example, an alkylation or esterification,
and, in a last step, a reduction with BH₃ × S(CH₃)₂ in tetrahydrofuran is carried out,
and, in the case of the pure enantiomers, an HPLC separation is carried out by customary methods,
and, if appropriate, the substituents listed above are introduced by customary methods and derivatized.

5. Pharmaceutical preparations which comprise, as active component, at least one compound according to any of Claims 1 to 3 in combination with at least one pharmaceutically acceptable essentially non-toxic vehicle or excipient.

6. Compounds according to any of Claims 1 to 3 for use as medicaments in the treatment of humans and animals.

7. Use of the compounds according to any of Claims 1 to 3 for preparing a medicament for the prevention and/or treatment of neurodegenerative disorders.

8. Use of the compounds according to any of Claims 1 to 3 for preparing a medicament for the prevention and/or treatment of cerebral ischaemias and craniocerebral trauma.

9. Use of the compounds according to any of Claims I to 3 for preparing a medicament for the treatment of states of pain, emesis, nausea, glaucoma, asthma, anorexia, convulsions, rheumatism, sedation and mobility disorders.

10. Use of the compounds according to any of Claims 1 to 3 for preparing a medicament for the treatment of bacterial or viral infections, autoimmune disorders, inflammatory or autoimmune disorders of the joints, of the bone and muscle apparatus, of internal and external organs, of the central nervous system, of the sensory organs and of the haemopoietic system in humans and animals.

11. Use of the compounds according to any of Claims 1 to 3 for preparing a medicament for the treatment of migraine and spasticity.

## Revendications

1. Composés de formule
R¹-O-G-OSO₂-R² (I),
dans laquelle
R¹ est un reste de formule 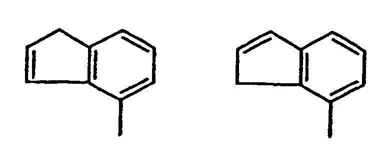 ou 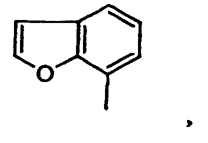
les systèmes cycliques indiqués ci-dessus étant éventuellement substitués avec un reste choisi dans le groupe des restes (alkoxy en C₁ à C₆)carbonyle ou alkyle en C₁ à C₈ substitué lui-même par un radical hydroxy,
G est un groupe phényle à deux liaisons, qui est éventuellement substitué avec un reste choisi dans le groupe des restes hydroxy, trifluorométhyle, fluoro, chloro, alkyle en C₁ à C₃, hydroxy(alkyle en C₁ à C₃) ou alkoxy en C₁ à C₃,
R² est un groupe alkyle en C₁ à C₈, qui est éventuellement substitué avec jusqu'à trois restes identiques ou différents choisis dans le groupe des restes fluoro, chloro, bromo, trifluorométhyle ou alkoxy en C₁ à C₄ à substituant trifluorométhyle,
et leurs sels acceptables du point de vue pharmaceutique.

2. Composés de formule (I) suivant la revendication 1,
formule dans laquelle
R¹ est un reste de formule 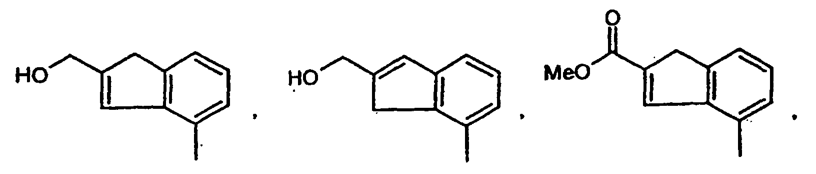 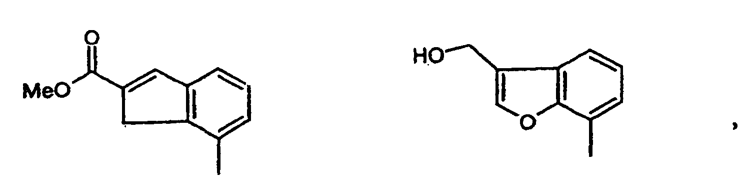
G est un groupe phényle à deux liaisons qui est éventuellement substitué par du fluor ou du chlore,
R² est un groupe alkyle en C₁ à C₄ qui est éventuellement substitué par du fluor ou un reste trifluorométhyle,
et leurs sels acceptables du point de vue pharmaceutique.

3. Composés suivant la revendication 1, choisis dans le groupe 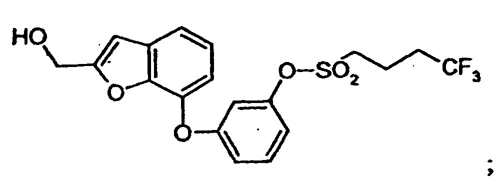 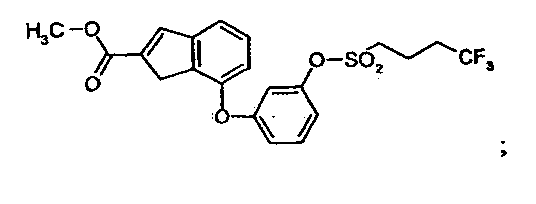 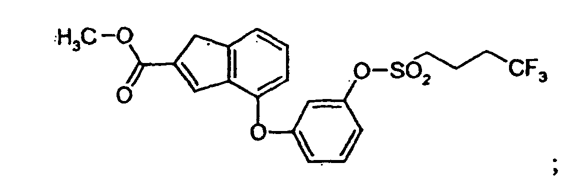 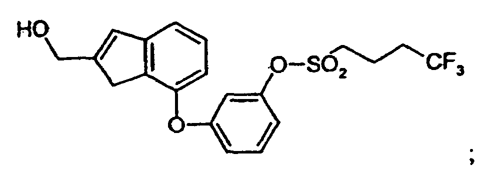 et

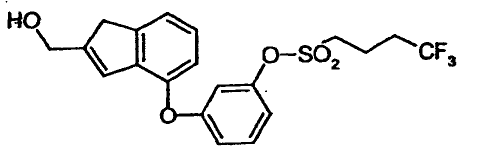

4. Procédé de production de nouveaux esters d'acide arylsulfonique suivant les revendications 1 à 3, **caractérisé en ce que**
[A] des composés de formule
R¹-O-G-OH (II),
dans laquelle
R¹ et G ont les définitions indiquées ci-dessus,
sont amenés à réagir avec des composés de formule
R³-SO₂-R² (III),
dans laquelle
R² a les définitions indiquées dans la revendication 1,
R³ est un halogène, de préférence le chlore ou l'iode,
pour obtenir des composés de formule (I), dans des solvants inertes, éventuellement en présence d'une base,
ou bien
[B] des composés de formule 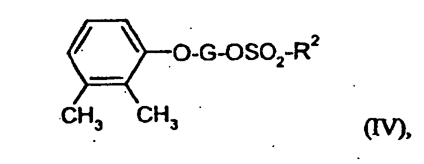
dans laquelle
G et R² ont les définitions indiquées ci-dessus,
sont transformés par bromation radicalaire, par exemple avec le N-bromosuccinimide, dans un solvant inerte, en composés de formule 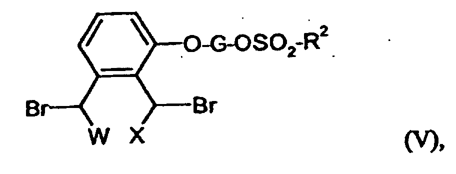 dans laquelle
G et R² ont les définitions indiquées ci-dessus,
et l'un des substituants W ou X représente le brome et l'autre représente l'hydrogène qui sont ensuite convertis par réaction avec des composés de formule
CH₂(CO₂R⁴)₂ (VI),
dans lesquels
R⁴ est un reste alkyle en C₁ à C₆
dans des solvants inertes, éventuellement en présence d'une base, en composés de formule
R⁴-O-G-OSO₂-R² (VII),
dans laquelle
G et R² ont les définitions indiquées ci-dessus et
R⁴ est un reste de formule 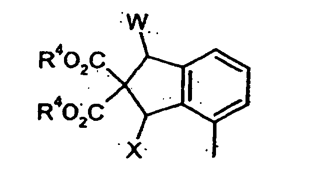 dans laquelle
R⁴, W et X ont les définitions indiquées ci-dessus, en composés de formule 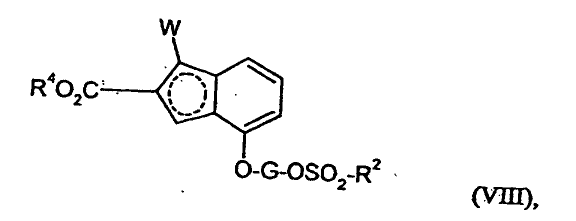 dans laquelle
G, R², W et R⁴ ont les définitions indiquées ci-dessus,
et on effectue finalement une réduction en la fonction méthylhydroxy,
ou bien
on effectue ensuite éventuellement des dérivatisations en fonction des substituants indiqués ci-dessus selon des modes opératoires usuels, par exemple par alkylation ou estérification,
et on effectue dans une dernière étape une réduction avec BH₃ x S(CH₃)₂ dans le tétrahydrofuranne,
et dans le cas des énantiomères purs, on effectue une séparation par CLHP selon des modes opératoires usuels,
et les substituants indiqués ci-dessus sont éventuellement introduits et dérivatisés selon des modes opératoires classiques.

5. Préparations pharmaceutiques, qui comprennent comme constituant actif au moins un composé selon l'une quelconque des revendications 1 à 3 conjointement avec au moins un support ou excipient essentiellement non toxique, acceptables du point de vue pharmaceutiques.

6. Composés suivant l'une quelconque des revendications 1 à 3, destinés à être utilisés comme médicament dans le traitement d'êtres humains et d'animaux.

7. Utilisation des composés suivant l'une quelconque des revendications 1 à 3 pour la préparation d'un médicament destiné au traitement préventif et/ou curatif de maladies neurodégénératives.

8. Utilisation des composés suivant l'une quelconque des revendications 1 à 3 pour la préparation d'un médicament destiné au traitement préventif et/ou curatif d'ischémies cérébrales et d'un traumatisme crâniocérébral.

9. Utilisation des composés suivant l'une quelconque des revendications 1 à 3 pour la préparation d'un médicament destiné au traitement d'états douloureux, de l'émèse, de la nausée, du glaucome, de l'asthme, de l'anorexie, de convulsions, de rhumatismes, de sédation et de troubles moteurs.

10. Utilisation des composés suivant l'une quelconque des revendications 1 à 3 pour la préparation d'un médicament destiné au traitement d'infections bactériennes ou virales, de maladies auto-immunes, de maladies à dépendance inflammatoires ou dépendance auto-immunologique des articulations de l'appareil osseux et musculaire, des organes internes et externes, du système nerveux central, des organes des sens et du système hématopoïétique chez l'homme et l'animal.

11. Utilisation des composés suivant l'une quelconque des revendications 1 à 3 pour la préparation d'un médicament destiné au traitement de la migraine et de la spasticité.
